# EUROPEAN PATENT SPECIFICATION

(11) **EP 4 577 130 B1**
(45) Date of publication and mention of the grant of the patent: **16.09.2026**
(21) Application number: 23768940.1
(22) Date of filing: 24.08.2023
(51) Int. Cl.: A61B 17/221

(54) **COLLAPSIBLE AND EXPANDABLE SUPPORT FRAMES FOR CATHETER TIPS**
ZUSAMMENKLAPPBARE UND EXPANDIERBARE STÜTZRAHMEN FÜR KATHETERSPITZEN
CADRES DE SUPPORT REPLIABLES ET EXPANSIBLES POUR POINTES DE CATHÉTER

(30) Priority: 25.08.2022 US 202217895308
(43) Date of publication of application: 02.07.2025
(73) Proprietor: Neuravi Limited, Galway H91 K5YD (IE)
(72) Inventor: KEATING, Karl, Galway, H91 K5YD (IE)
(74) Representative: Carpmaels & Ransford LLP
(86) International application number: PCT/IB2023/058405
(87) International publication number: WO 2024/042481

(56) References cited:
- AU-A1- 2020 407 593
- CN-A- 113 349 880
- CN-A- 113 813 489
- US-A1- 2016 192 953

## Description

### FIELD

The present invention generally relates to devices for removing acute blockages from blood vessels during intravascular medical treatments. More specifically, the present invention relates to retrieval catheters with expandable tips into which an object or objects can be retrieved.

### BACKGROUND

Clot retrieval aspiration catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and highly tortuous. Traditional devices are often either too large in profile, lack the deliverability and flexibility needed to navigate particularly tortuous vessels, or are ineffective at removing a clot when delivered to the target site.

Many existing designs for aspiration retrieval catheters are often restricted to, for example, inner diameters of 6Fr or between approximately 0.068 - 0.074 inches. Larger sizes require a larger guide or sheath to be used, which then necessitates a larger femoral access hole to close. Most physicians would prefer to use an 8Fr guide / 6Fr sheath combination, and few would be comfortable going beyond a 9Fr guide / 7Fr sheath combination. This means that once at the target site, a clot can often be larger in size than the inner diameter of the aspiration catheter and must otherwise be immediately compressed to enter the catheter mouth. This compression can lead to bunching up and subsequent shearing of the clot during retrieval. Firm, fibrin-rich clots can also become lodged in the fixed-mouth tip of these catheters making them more difficult to extract. This lodging can also result in shearing where softer portions break away from firmer regions of the clot. (6Fr = 2.0 mm, 7Fr = 2.3mm, 8Fr = 2.7mm, 9Fr = 3mm).

Small diameters and fixed tip sizes are also less efficient at directing the aspiration necessary to remove blood and thrombus material during the procedure. Fixed tip sizes can cause a clot to shear or break apart as the clot enters the tip opening. The suction must be strong enough such that any fragmentation that may occur as a result of aspiration or the use of a mechanical thrombectomy device can be held stationary so that fragments cannot migrate and occlude distal vessels. However, when aspirating with a fixed-mouth catheter, a significant portion of the aspiration flow ends up coming from vessel fluid proximal to the tip of the catheter, where there is no clot, because the diameter of the funnel catheter is smaller than that of the vessel. This significantly reduces aspiration efficiency, lowering the success rate of clot removal.

Any catheter design attempting to overcome these challenges with an expanding distal tip or structure would need to have the strength to extract the clot and exert a steady radial force in the expanded state. The same structure would also need to be sufficiently flexible and elastic to survive the severe mechanical strains imparted when navigating tortuous vasculature when in a collapsed state.

As a result, there remains a need for improved catheter designs attempting to overcome the above-mentioned design challenges. The present designs are aimed at providing an improved retrieval catheter with an expansile tip section and methods for using such a catheter capable of improved performance.

CN113349880A describes a system that has an outer catheter and an inner aspirating clot retrieval catheter having an expansile distal tip for flow restriction, improved aspiration efficiency, and a large mouth into which a clot or other obstructions can be retrieved. The clot retrieval catheter can have a support tube proximal of the tip. The expansile tip can be a strut framework, and a flexible, low-modulus cover is disposed around at least a portion of the tip strut framework and the proximal support tube. The distal end of the tip can be encapsulated by a low-friction elastomeric lip for atraumatic contact with the walls of a blood vessel. The tip has a collapsed delivery configuration and expands radially into a deployed configuration.

US2016192953A1 describes a clot retrieval device for removing clot from a blood vessel comprises an inner elongate body and an outer elongate body at least partially overlying the inner elongate body The device also comprises an elongate member or shaft having a proximal end which extends exterior of a patient so that a user can retrieve the stent-basket device and captured clot by retracting the shaft The outer elongate body and the inner elongate body are connected to the distal end of the shaft and are expandable relative to the shaft from a collapsed delivery configuration to an expanded deployed configuration. The outer elongate body is expandable relative to the inner elongate body to a radial extent which is greater than the radial extent of the inner body in the deployed configuration.

AU2020407593A1 describes systems and methods for the intravascular treatment of clot material within a blood vessel of a human patient. A system includes a coring element for coring and separating the clot material. The coring element can comprise a unitary structure having a first region, a second region, a third region, and a fourth region. The first region is adjacent to a proximal portion of the unitary structure and includes a first mouth configured to core and separate the clot material. The second region is distal of the first region, generally tubular, and includes a first plurality of interconnected struts. The third region is distal of the second region and includes a second mouth configured to core and separate the vascular thrombus. The fourth region is distal of the third region, generally tubular, and includes a second plurality of interconnected struts.

CN113813489A describes an expandable mouth for a catheter that is capable of locally restricting flow and providing a large mouth opening that provides sufficient radial force while having flexibility to reach and retrieve occluded clots. The expandable mouth may have a frame having a collapsed state, so that the clot retrieval catheter may be a compatible thin outer catheter and has an expanded state for contacting the vessel wall and sealing or limiting proximal flow. The frame may have one or more support arms and a distal support cuff, which may have narrowed sections, undulations, closed pores, and other flexibility enhancing features. Alternatively, the frame may be a grid having an array of closed pores. The expandable mouth may be characterized by a membrane cover disposed around the support frame.

### SUMMARY

Claims 1 and 8 define the invention, dependent claims disclose embodiments. It is an object of the present designs to provide devices and methods to meet the above-stated needs. The designs can be for a clot retrieval catheter capable of removing a clot from cerebral arteries in patients suffering from AIS, from coronary native or graft vessels in patients suffering from MI, and from pulmonary arteries in patients suffering from PE and from other peripheral arterial and venous vessels in which a clot is causing an occlusion.

One example of the present disclosure provides a catheter tip. The catheter tip can include a support frame. The support frame can include a longitudinal axis, a collapsed delivery configuration, and an expanded deployed configuration. The support frame can include one or more profiled spine members that extend along the longitudinal axis. The one or more profiled spine members can connect a distal most rib with a proximal most rib. The support frame can include a plurality of ribs that extend from the one or more profiled spine members forming a circumference of the support frame. The support frame can include one or more connector struts each connecting respective adjacent ribs of the plurality of ribs. The plurality of connector struts can be offset from the one or more profiled spine members. The support frame can further include a collapsed inner diameter in the collapsed delivery configuration and a larger expanded inner diameter in the expanded deployed configuration when the support frame is placed in axial compression.

In some examples, the support frame is configured to expand from the collapsed inner diameter to the expanded inner diameter when impinged by an ingested clot.

In some examples, the support frame can further include a proximal collar at the proximal end of the support frame.

In some examples, the plurality of ribs can each include a curvilinear, non-planar profile.

In some examples, each of the one or more connector struts are substantially parallel to the one or more profiled spine members.

In some examples, each of the one or more connector struts can be spaced approximately around the circumference of the support frame from each of the one or more profiled spine members.

In some examples, the one or more profiled spine members can include two profiled spine members.

In some examples, two or more ribs of the plurality of ribs are unconnected by the one or more connector struts.

In another aspect, a catheter tip is disclosed. The catheter tip can include a support frame. The support frame can include a longitudinal axis, a collapsed delivery configuration, and an expanded deployed configuration. The support frame can include a connector ribs extending around a circumference of the support frame in a first direction. The support frame can include a plurality of offset ribs extending from the connector rib around the circumference of the support frame in the second direction. The support frame can include one or more connector members each connecting respective ribs of the plurality of offset ribs. The support frame can further include a collapsed inner diameter in the collapsed delivery configuration and a larger expanded inner diameter in the expanded deployed configuration when the support frame is placed in axial compression.

In some examples, the one or more connector members can further include one or more connector struts.

In some examples, the one or more connector struts are substantially parallel to the longitudinal axis.

In some examples, the one or more connector struts connect respective adjacent ribs of the plurality of offset ribs.

In some examples, the one or more connector members can further include one or more compression cells.

In some examples, the one or more compression cells can include a generally diamond-shaped pattern.

In some examples, the one or more compression cells can include a generally ovular-shaped pattern.

In some examples, the first direction and the second direction are approximately orthogonal.

In another aspect, a catheter tip is disclosed. The catheter tip can include a support frame. The support frame can include a longitudinal axis, a collapsed delivery configuration, and an expanded deployed configuration. The support frame can include a first connector rib extending from a proximal end of the support frame around a circumference of the support frame. The support frame can include a first plurality of offset ribs extending from the first connector rib around the circumference of the support frame. The support frame can include a second connector rib extending from the proximal end of the support frame and around circumference of the support frame. The support frame can include a second plurality of offset ribs extending from the second connector rib around the circumference of the support frame. The support frame can include a collapsed inner diameter in the collapsed delivery configuration and a larger expanded inner diameter in the expanded deployed configuration when the support frame is placed in axial compression.

In some examples, the catheter tip can include one or more connector struts each connecting respective adjacent ribs of the first plurality of offset ribs or the second plurality of offset ribs.

In another aspect, a catheter tip is disclosed. The catheter tip can include a support frame including a longitudinal axis, a collapsed delivery configuration, and an expanded deployed configuration. The support frame can include a plurality of struts extending form the proximal end of the support frame. The plurality of struts can form a lattice structure. The support frame can include a collapsed inner diameter in the collapsed delivery configuration and a larger expanded inner diameter in the expanded deployed configuration when the support frame is placed in axial compression.

In some examples, the lattice structure can further include a plurality of proximal cells approximate the proximal end that has a proximal cell angle and a plurality of distal cells approximate a distal end of the support frame having a distal cell angle. In some examples, the proximal cell angle can be greater than the distal cell angle.

In some examples, the plurality of struts form a generally V-shaped void approximate the proximal end of the support frame. The generally V-shaped void can include a vertex extending toward the distal end of the support frame.

In another aspect, a catheter tip is disclosed. The catheter tip can include a support frame that includes a longitudinal axis, a collapsed delivery configuration, and an expanded deployed configuration. The support frame can include one or more helical spines extending from a proximal end of the support frame. The support frame can include one or more offset ribs extending from each helical spine of the one or more helical spines. The support frame can include a collapsed inner diameter in the collapsed delivery configuration and a larger expanded inner diameter in the expanded deployed configuration when the support frame is placed in compression.

In some examples, the one or more offset ribs extend around a circumference of the support frame. The one or more offset ribs can be substantially orthogonal to the longitudinal axis.

In some examples, the one or more helical spines can include one helical spine.

In some examples, the one or more helical spines can include four helical spines.

In some examples, the one or more helical spines can include a number of spines between one helical spine and twelve helical spines.

Other aspects of the present disclosure will become apparent upon reviewing the following detailed description in conjunction with the accompanying figures. Additional features or manufacturing and use steps can be included as would be appreciated and understood by persons skilled in the pertinent art.

### BRIEF DESCRIPTION OF THE DRAWINGS

The above and further aspects of this disclosure are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation. It is expected that those of skill in the pertinent art can conceive of and combine elements from multiple figures to better suit the needs of the user.
FIG. 1 is a perspective view of a collapsible and expandable support frame having a plurality of ribs in an expanded configuration, according to aspects of the present invention.
FIG. 2 is a perspective view of a collapsible and expandable support frame having a plurality of offset ribs in an expanded configuration, according to aspects of the present invention.
FIG. 3 is a perspective view of a collapsible and expandable support frame having a plurality of offset ribs and generally diamond-shaped compression cells in an expanded configuration, according to aspects of the present invention.
FIG. 4 is a perspective view of a collapsible and expandable support frame having a plurality of offset ribs and generally ovular-shaped compression cells in an expanded configuration, according to aspects of the present invention.
FIG. 5 is a perspective view of a collapsible and expandable support frame having a plurality of offset ribs in an expanded configuration connected with one or more connector struts, according to aspects of the present invention.
FIG. 6 is a perspective view of a collapsible and expandable support frame having a proximal cell and distal cell in an expanded configuration.
FIG. 7 is a perspective view of a shortened collapsible and expandable support frame having a proximal cell and distal cell in an expanded configuration.
FIG. 8 is a perspective view of a collapsible and expandable support frame having a proximal cell and distal cell in an expanded configuration having a V-shaped void.
FIG. 9 is a perspective view of a collapsible and expandable support frame having helical struts in an expanded configuration.
FIG. 10 is a perspective view of a shortened collapsible and expandable support frame having helical struts an expanded configuration.
FIG. 11 is a perspective view of a shortened collapsible and expandable support frame having helical spines an expanded configuration.
FIG. 12A is a cross-sectional view of a collapsed inner diameter of an example support frame, according to aspects of the present invention.
FIG. 12B is a cross-sectional view of an expanded inner diameter of an example support frame, according to aspects of the present invention.
FIG. 13 is a diagram of a clot retrieval catheter tip in accordance with the present invention with an expandable support frame being advanced through the vasculature.
FIGS. 14A-14C are illustrations of example treatment steps that can be performed using a collapsible and expandable catheter tip support frame.
FIGS. 15A-15C are illustrations of example treatment steps that can be performed using a collapsible and expandable catheter tip support frame.

### DETAILED DESCRIPTION

Specific examples of the present invention are now described in detail with reference to the Figures, where identical reference numbers indicate elements which are functionally similar or identical. The examples address many of the deficiencies associated with traditional clot retrieval aspiration catheters, such as poor or inaccurate deployment to a target site and ineffective clot removal.

The designs herein, illustrating various configurations of catheter tip support frames, can be incorporated into an aspiration clot retrieval catheter with a membrane cover, proximal shaft, large bore lumen, and a distal low shear tip (LST) that can expand to a diameter larger than the nominal diameter when it interacts with an ingested clot or stentriever. The designs herein can also be pre-expanded and heat set to incorporate into a collapsible super bore (CSB) catheter with a membrane cover and proximal shaft to provide a catheter that collapses for delivery through a guide catheter and expands when exiting the guide catheter to be advanced to a target vessel for aspiration of a clot.

The designs herein can have a proximal elongate body for the shaft of the catheter, and a distal tip with an expanding inner frame to give the tip atraumatic properties. That is, the expanding inner frame is capable of easily and repeatedly collapsing for delivery and expanding locally either under loading from the clot (when used in conjunction with an LST catheter), or by being heat set (when used in conjunction with a CSB catheter), thereby enabling the catheter tip to expand beyond the nominal diameter to ingest a clot. The expanding inner frame can have a proximal ring for attaching to a braided catheter shaft, and can have an offset mouth allowing for a larger opening for clot retrieval and reduced stiffness for easier expansion. This management of the clot during ingestion can significantly reduce shearing of the clot. The catheter's design can be sufficiently flexible to navigate highly tortuous areas of the anatomy and be able to recover its shape to maintain the inner diameter of the lumen when displaced in a vessel. Alternatively, the designs herein can be formed integrally with a laser cut shaft support structure or otherwise attached to a laser cut shaft support structure.

This innovation of utilizing the clot itself to expand the tip section as needed allows for much improved clot handling and less shearing over traditional designs. The nominal, non-expanded outer diameter maximizes distal access reach like a standard fixed-mouth catheter. Once a clot is subsequently ingested, accommodating stiff, fibrin-rich portions of the clot through additional radial expansion can gradually compress the clot such that there is significantly less clot shearing than catheters that lack this capability. Further, the conformable nature of the tip allows it to be advanced atraumatically past calcified lesions without dislodging plaque material.

Accessing the various vessels within the vascular system, whether they are coronary, pulmonary, or cerebral vessels, involves well-known procedural steps and the use of a number of conventional, commercially-available accessory products. These products, such as angiographic materials, mechanical thrombectomy devices, microcatheters, and guidewires are widely used in laboratory and medical procedures. When these products are employed in conjunction with the devices of this invention in the description below, their function and exact constitution are not described in detail. Additionally, while the description is in many cases in the context of thrombectomy treatments in intercranial arteries, the disclosure may be adapted for other procedures and in other body passageways as well.

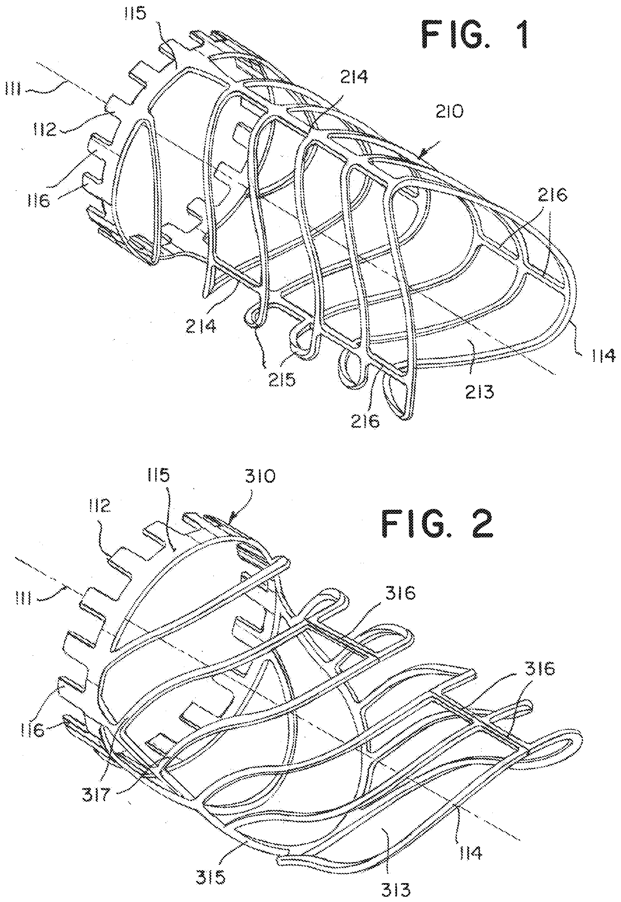

Turning to the figures, FIG. 1 illustrate an example collapsible and expandable support frame 210 for use in a clot retrieval catheter tip 100. When used in conjunction with an LST catheter, support frame 210 can be manufactured using super elastic nickel titanium or Nitinol (NiTi), shape memory NiTi, or stainless steel. While shape memory material is not required for an LST catheter, it can provide an added benefit of enabling the support frame 210 to recover its shape if distorted during use, or if support frame 210 is expanded by a clot during a first retrieval pass and needs to be easily recovered to track back through a guide sheath for making a second retrieval pass. When used in conjunction with a CSB catheter, shape memory material enables support frame 210 to self-expand when it exits the distal end of the catheter tip 100 and approaches the clot for retrieval. The shape memory alloy used can include an Austenite finish temperature less than approximately 30 degrees Celsius.

Support frame 210 can be manufactured by taking raw tubing, of a material as discussed above, and laser cutting the material to produce the desired configuration of support frame 210, as will be described further below. The raw tubing can have an outer diameter of approximately 2.00 millimeters, a wall thickness of approximately 0.05 millimeters, and an inner diameter of approximately 1.90 millimeters. Support frame 210 can also be manufactured to have light electropolishing or other such finish. A matte finish can provide a benefit of enhancing adhesion to a polymer catheter jacket.

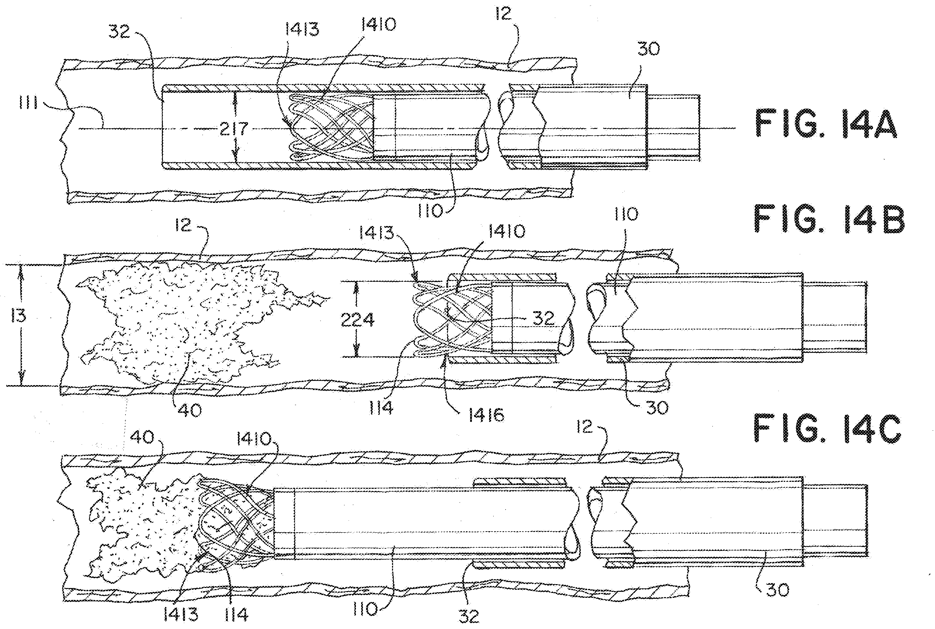

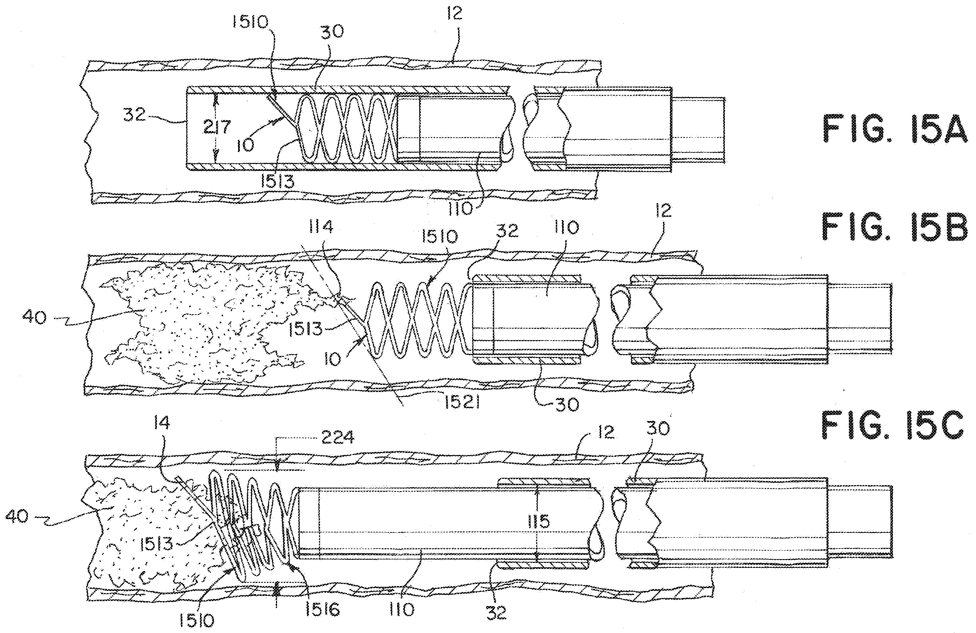

The support frame can include a longitudinal axis 111, a collapsed delivery configuration (FIG. 14A, FIG. 15A, or FIG. 15B), and an expanded deployed configuration (FIGS. 14B, FIG. 14C, or FIG. 15C). The support frame 210 can include one or more profiled spines 214 that can be spaced apart by 180 degrees about the longitudinal axis 111. Ribs 215 can extend from the one or more profiled spines 214 such that the one or more profiled spines 214 connect a distal most rib with a proximal most rib. Ribs 215 can form a circumference of the support frame 210. A distal end 114 of the support frame can include a mouth 213 that is shaped by the circumference of the support frame shaped by the ribs 215. A proximal end 112 of the support frame 210 can include a collar 115 having a plurality of collar prongs 116 that can assist with connecting a catheter tip 100 to an elongate body of a catheter delivery system by providing additional surface area to aid in welding or bonding the catheter tip 100 to a catheter system, according to some embodiments. Some ribs 215 can be connected by one or more connector struts 216. Connector struts 216 can be rotated by approximately 90 degrees about the longitudinal axis 111 with respect to the one or more profiled spines 214. Such configuration of connector struts 216 can improve the crush resistance of the expanded tip when tracking through tortuous vasculature as catheter tip 100 is positioned for clot retrieval.

FIG. 2 illustrates another example expandable support frame 310 for use in a clot retrieval catheter tip 100. Support frame 310 can include one or more features that are the same as or similar to those described above with respect to support frame 210. Further, the manufacturing of support frame 310 can be the same as or similar to that of support frame 210, as discussed above. Support frame 310 can also be manufactured to be electropolished, pickled to roughen the surface of support frame 310, and/or heated to have an oxide layer finish.

Support frame 310 can include longitudinal axis, a collapsed delivery configuration, and an expanded deployed configuration. Support frame 310 can include a connector rib 315 that extends around a circumference of the support frame in a first direction. Extending from the connector rib 315 can be a plurality of offset ribs that extend around the circumference of the support frame 310 in a second direction. In some examples, the first direction and the second direction can be approximately orthogonal to one another. Support frame 310 can also include one or more connector struts 316. The one or more connector struts 316 can connect respective ribs of the plurality of offset ribs 317. The one or more connector struts 316 can extend approximately parallel to the longitudinal axis 111. The connection between the offset ribs 317 and connector ribs 315 can facilitate the support frame 310 compressing smoothly when pressing into a wall of vasculature when navigating tortuous anatomy.

The support frame 310 can also include a proximal collar 115 at its proximal end 112. The proximal collar 115 can be used for attaching the support frame 310 to a braided shaft of a clot retrieval catheter. The proximal collar 115 can include a collar prongs 116 which can aid in the attachment process of support frame 310 to a braided shaft of clot retrieval catheter.

Support frame 310 can also include mouth 313 at a distal end 114. Similar to support frame 210, support frame 310 can include a collapsed inner diameter when in the collapsed delivery configuration, and a larger inner diameter in the expanded deployed configuration when the support frame 310 is placed in compression. Used in conjunction with either an LST or CSB catheter, when support frame 310 is in its expanded deployed configuration, its maximum outer diameter can be less than an inner diameter 13 of a target vessel 12 at a treatment site, such that support frame 310 can advance distally toward clot 40 independently from and without sealing to the vessel 12.

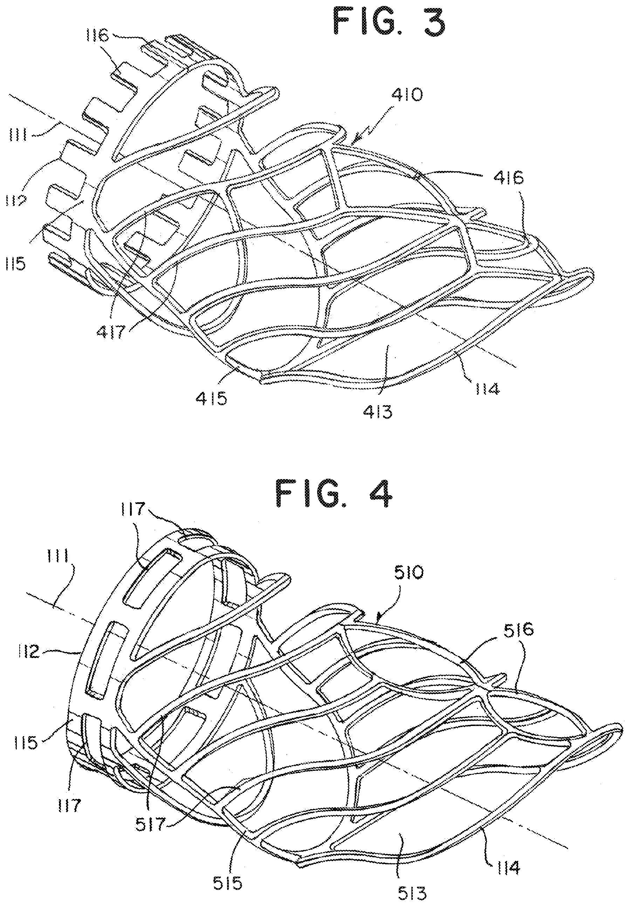

FIG. 3 illustrates another example expandable support frame 410 for use in a clot retrieval catheter tip 100. Support frame 410 can include one or more features that are the same as or similar to those described above with respect to support frame 310. Further, the manufacturing of support frame 410 can be the same as or similar to that of support frame 310, as discussed above. Support frame 410 can also be manufactured to be electropolished, pickled to roughen the surface of support frame 410, and/or heated to have an oxide layer finish.

Support frame 410 can be substantially similar to support frame 310, but in place of the connector struts 317 of support frame 310, support frame 410 can include a generally diamond-shaped compression cells 416. Generally diamond-shaped compression cells 416 can connect respective offset ribs. Unlike connector struts 317, compression cells 416 can connect a greater number of offset ribs 417. Rather than connecting two offset ribs 317 like in support frame 310, compression cell 416 can connect three or more offset ribs 417 as shown in FIG. 3. Compression cells 416 can allow for smoother compression of support frame 410 over a greater number of offset ribs 417 while facilitating lateral flex to the support frame 410.

Similar to support frame 210, support frame 410 can include a collapsed inner diameter when in the collapsed delivery configuration, and a larger inner diameter in the expanded deployed configuration when the support frame 410 is placed in compression. Used in conjunction with either an LST or CSB catheter, when support frame 410 is in its expanded deployed configuration, its maximum outer diameter can be less than an inner diameter 13 of a target vessel 12 at a treatment site, such that support frame 310 can advance distally toward clot 40 independently from and without sealing to the vessel 12.

FIG. 4 illustrates another example expandable support frame 510 for use in a clot retrieval catheter tip 100. Support frame 510 can include one or more features that are the same as or similar to those described above with respect to support frame 410. Further, the manufacturing of support frame 510 can be the same as or similar to that of support frame 410, as discussed above. Support frame 510 can also be manufactured to be electropolished, pickled to roughen the surface of support frame 510, and/or heated to have an oxide layer finish.

Support frame 510 can be substantially similar to support frame 410, but in place of the generally diamond-shaped compression cells 416 of support frame 410, support frame 510 can include a generally ovular-shaped compression cells 516. Generally ovular-shaped compression cells 516 can connect respective offset ribs. Similar to compression cells 416, compression cells 516 can connect a greater number of offset ribs 517. That is, compression cells 516 can connect three or more offset ribs 517 as shown in FIG. 4. Compression cells 516 can allow for smoother compression of support frame 510 over a greater number of offset ribs 517 while facilitating lateral flex to the support frame 510.

Similar to support frame 210, support frame 510 can include a collapsed inner diameter when in the collapsed delivery configuration, and a larger inner diameter in the expanded deployed configuration when the support frame 510 is placed in compression. Used in conjunction with either an LST or CSB catheter, when support frame 510 is in its expanded deployed configuration, its maximum outer diameter can be less than an inner diameter 13 of a target vessel 12 at a treatment site, such that support frame 510 can advance distally toward clot 40 independently from and without sealing to the vessel 12.

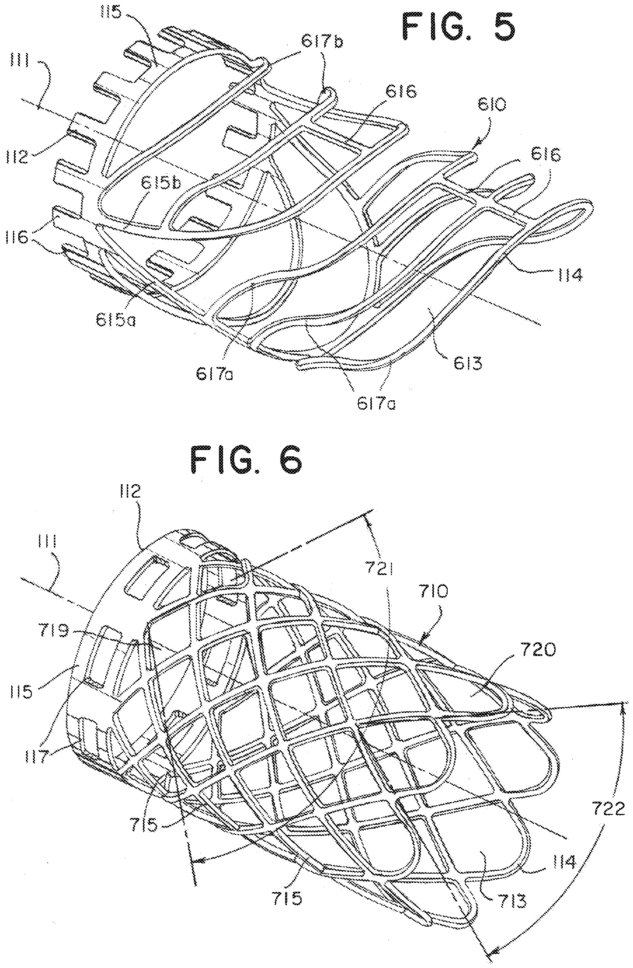

FIG. 5 illustrates another example expandable support frame 610 for use in a clot retrieval catheter tip 100. Support frame 610 can include one or more features that are the same as or similar to those described above with respect to support frame 210. Further, the manufacturing of support frame 610 can be the same as or similar to that of support frame 210, as discussed above. Support frame 610 can also be manufactured to be electropolished, pickled to roughen the surface of support frame 610, and/or heated to have an oxide layer finish.

The support frame 610 can include a longitudinal axis 111, a collapsed delivery configuration, and an expanded deployed configuration. Support frame 610 can include a first connector rib 615a that extends from a proximal end 112 of the support frame and around a circumference of the support frame 610. A first plurality of offset ribs 617a can extend from the first connector rib 615a and around the circumference of the support frame 610. Support frame 610 can also include a second connector rib 615 that extends from the proximal end 112 of the support frame and around the circumference of the support frame 610. Extending from the second connector rib 615b can be a second plurality of offset ribs 617b that extend around the circumference of the support frame 610. The connection between the offset ribs 617a, 617b and the connector ribs 615a, 615b can allow the support frame 610 to compress smoothly when pressing into a wall of a vessel while navigating tortuous anatomy. In some examples, support frame 610 can include one or more connector struts 616 that each can connect respective adjacent ribs of the first plurality of offset ribs 617a or the second plurality of offset ribs 617b. Connector struts 616 can improve the crush resistance of the expanded tip when tracking through tortuous vasculature as catheter tip 100 is positioned for clot retrieval.

A proximal end 112 of the support frame 610 can include a collar 115 having a plurality of collar prongs 116 that can assist with connecting a catheter tip 100 to an elongate body of a catheter delivery system by providing additional surface area to aid in welding the catheter tip 100 to a catheter system, according to some embodiments.

FIG. 6 illustrates another example expandable support frame 710 for use in a clot retrieval catheter tip 100. Support frame 710 can include one or more features that are the same as or similar to those described above with respect to support frame 210. Further, the manufacturing of support frame 710 can be the same as or similar to that of support frame 210, as discussed above. Support frame 710 can also be manufactured to be electropolished, pickled to roughen the surface of support frame 710, and/or heated to have an oxide layer finish.

Support frame 710 can include a longitudinal axis 111, a collapsed delivery configuration, and an expanded deployed configuration. The support frame 710 can include a plurality of struts 715 that extend from proximal end 112 of the support frame 710. The plurality of struts 715 can intersect one another to form a lattice structure. The intersecting struts 715 of support frame 710 can form cells. In some examples, the support frame 710 can include proximal cells 719 having a proximal cell angle 721 and distal cells 720 having distal cell angles 722. When in a collapsed configuration, the proximal cell angle 721 of proximal cells 719 can be greater than the distal cell angles 722 of distal cells 720. The distal cell angle 722, while in the collapsed configuration, are effective to reduce radial force on the support frame 710 such that tracking the catheter tip 100 through an outer guide sheath is facilitated. When support frame 710 expands to the expanded deployed configuration, proximal cell angles 721 of proximal cells may not substantially change, while distal cell angles 722 of distal cells 720 can increase in the expanded deployed configuration. The expansion of distal cells angles of distal cells 720 can improve crush resistance while catheter tip 100 using support frame 710 is used to aspirate a clot and/or while catheter tip 100 is tracking through tortuous vasculature while approaching a clot to be aspirated.

The distal cell angle 722 in the collapsed configuration can be in the range of 20 to 130 degrees, more preferably in the range of 30 to 90 degrees, even more preferably in the range of 30 to 60 degrees. Lower angles can allow the tip to expand to greater diameters than higher angles while higher angles can provide the tip with higher crush resistance for a given strut width. The proximal angle 721 can be in the range of 20 to 160 degrees, more preferably in the range of 30 to 120 degrees, even more preferably in the range of 60 to 90 degrees. Having the proximal angle 721 higher than the distal angle 722 can facilitate a more gradual expansion of the tip during clot ingestion. When expanded, the angles 721, 722 will depend on the diameter the tip is expanded to during clot ingestion for the LST embodiment. For the CSB embodiment, the distal cell angle 722 should be set such that the tip has sufficient crush resistance while having low enough radial force such that the tip can be collapsed and advanced through an outer guide catheter - for this purpose, the distal cell angle 722 can be in the range of 60 to 130 degrees, more preferably in the range of 90 to 120 degrees, even more preferably in the range of 100 to 110 degrees. For the CSB the proximal cell angle 721 can be lower or higher than that of the distal cell angle 722. The proximal angle 721 can be in the range of 20 to 160 degrees, more preferably in the range of 30 to 120 degrees, even more preferably in the range of 60 to 90 degrees. Having the proximal cell angle 721 lower than that of the distal angle 722 can provide more push when collapsing the expanded portion for delivery while having the proximal angle 721 higher than the distal angle 722 give more gradual expansion/compression of the tip during delivery/collapse.

A proximal end 112 of the support frame 710 can include a wide collar 115 having a plurality collar cavities 117. Wide collar 115 is provided to give sufficient material for welding to a braided structure of an elongate body of a catheter delivery system by providing additional surface area to aid in welding the catheter tip 100 to a catheter system. Collar cavities 117 can be provided to allow materials to reflow during a lamination process to improve adhesion between the support frame 710 and the elongate body of a catheter delivery system. The collar cavities 117 can also be effective to prevent excess material from bunching up over collar 115 in order to keep the outer diameter profile of catheter tip 100 low and even throughout the structure. The cavities 117 may also be used as wells to guide adhesive should a braided structure be positioned over or under the collar 115.

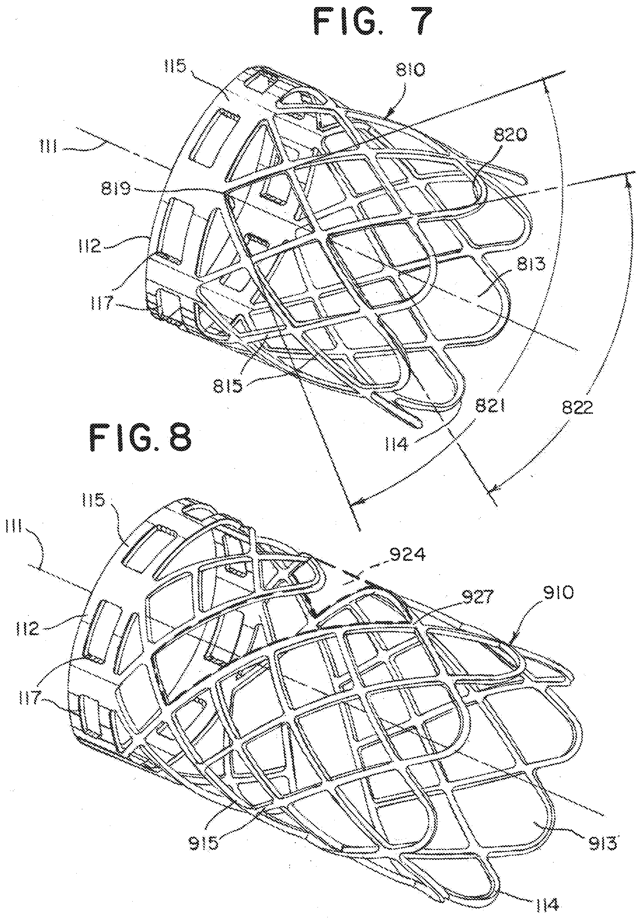

FIG. 7 illustrates another example expandable support frame 810 for use in a clot retrieval catheter tip 100. Support frame 810 can include one or more features that are the same as or similar to those described above with respect to support frame 710. Further, the manufacturing of support frame 810 can be the same as or similar to that of support frame 710, as discussed above. Support frame 810 can also be manufactured to be electropolished, pickled to roughen the surface of support frame 810, and/or heated to have an oxide layer finish.

Support frame 810 can include a longitudinal axis 111, a collapsed delivery configuration, and an expanded deployed configuration. The support frame 810 can include a plurality of struts 815 that extend from proximal end 112 of the support frame 810. The plurality of struts 815 can intersect one another to form a lattice structure. The intersecting struts 815 of support frame 810 can form cells. In some examples, the support frame 810 can include proximal cells 819 having a proximal cell angle 821 and distal cells 820 having distal cell angles 822. When in a collapsed configuration, the proximal cell angle 821 of proximal cells 819 can be greater than the distal cell angles 822 of distal cells 820. The distal cell angles 822, while in the collapsed configuration, are effective to reduce radial force on the support frame 810 such that tracking the catheter tip 100 through an outer guide sheath is facilitated. When support frame 810 expands to the expanded deployed configuration, proximal cell angles 821 of proximal cells may not substantially change, while distal cell angles 822 of distal cells 820 can increase in the expanded deployed configuration. The expansion of distal cells angles of distal cells 820 can improve crush resistance while catheter tip 100 using support frame 810 is used to aspirate a clot and/or while catheter tip 100 is tracking through tortuous vasculature while approaching a clot to be aspirated.

The distal cell angle 822 and the proximal cell angle 821 can be configured to provide properties of an LST catheter or a CSB catheter as similar to the cell angles 721, 722 illustrated in FIG. 6. The distal cell angle 822 and the proximal cell angle 821 can have measurements like those of the cell angles 721, 722 illustrated in FIG. 6. The distal cell angle 822 in the collapsed configuration can be in the range of 20 to 130 degrees, more preferably in the range of 30 to 90 degrees, even more preferably in the range of 30 to 60 degrees. Lower angles can allow the tip to expand to greater diameters than higher angles while higher angles can provide the tip with higher crush resistance for a given strut width. The proximal angle 821 can be in the range of 20 to 160 degrees, more preferably in the range of 30 to 120 degrees, even more preferably in the range of 60 to 90 degrees. Having the proximal angle 821 higher than the distal angle 822 can facilitate a more gradual expansion of the tip during clot ingestion. When expanded, the angles 821, 822 will depend on the diameter the tip is expanded to during clot ingestion for the LST embodiment. For the CSB embodiment, the distal cell angle 822 should be set such that the tip has sufficient crush resistance while having low enough radial force such that the tip can be collapsed and advanced through an outer guide catheter - for this purpose, the distal cell angle 822 can be in the range of 60 to 130 degrees, more preferably in the range of 90 to 120 degrees, even more preferably in the range of 100 to 110 degrees. For the CSB the proximal cell angle 821 can be lower or higher than that of the distal cell angle 822. The proximal angle 821 can be in the range of 20 to 160 degrees, more preferably in the range of 30 to 120 degrees, even more preferably in the range of 60 to 90 degrees. Having the proximal cell angle 821 lower than that of the distal angle 822 can provide more push when collapsing the expanded portion for delivery while having the proximal angle 821 higher than the distal angle 822 give more gradual expansion/compression of the tip during delivery/collapse.

A proximal end 112 of the support frame 810 can include a wide collar 115 having a plurality collar cavities 117. Wide collar 115 is provided to give sufficient material for welding to a braided structure of an elongate body of a catheter delivery system by providing additional surface area to aid in welding the catheter tip 100 to a catheter system. Collar cavities 117 can be provided to allow materials to reflow during a lamination process to improve adhesion between the support frame 810 and the elongate body of a catheter delivery system. The collar cavities 117 can also be effective to prevent excess material from bunching up over collar 115 in order to keep the outer diameter profile of catheter tip 100 low and even throughout the structure.

Support frame 810 can be similar to support frame 710, except that the support frame 810 can be reduced in axial length along the longitudinal axis 111 as compared to support frame 710. A reduced length of support frame 810 can be effective to improve maneuverability of support frame 810 as it navigates tortuous vasculature.

FIG. 8 illustrates another example expandable support frame 910 for use in a clot retrieval catheter tip 100. Support frame 910 can include one or more features that are the same as or similar to those described above with respect to support frame 710. Further, the manufacturing of support frame 910 can be the same as or similar to that of support frame 710, as discussed above. Support frame 910 can also be manufactured to be electropolished, pickled to roughen the surface of support frame 910, and/or heated to have an oxide layer finish.

A proximal end 112 of the support frame 910 can include a wide collar 115 having a plurality collar cavities 117. Wide collar 115 is provided to give sufficient material for welding to a braided structure of an elongate body of a catheter delivery system by providing additional surface area to aid in welding the catheter tip 100 to a catheter system. Collar cavities 117 can be provided to allow materials to reflow during a lamination process to improve adhesion between the support frame 910 and the elongate body of a catheter delivery system. The collar cavities 117 can also be effective to prevent excess material from bunching up over collar 115 in order to keep the outer diameter profile of catheter tip 100 low and even throughout the structure.

Support frame 910 can include a longitudinal axis 111, a collapsed delivery configuration, and an expanded deployed configuration. The support frame 910 can include a plurality of struts 915 that extend from proximal end 112 of the support frame 910. The plurality of struts 915 can intersect one another to form a lattice structure. The intersecting struts 915 of support frame 910 can form cells. In some examples, the support frame 910 can also include a V-cell 924 that can be a V-shaped void in the lattice formed from the intersecting struts 915. The V-cell 924 can be positioned such that a V-cell vertex 927 of the V-cell 924 is positioned facing towards the distal end 114 of the support frame 910. The V-cell 924 can be positioned approximate the proximal end 112 and can be effective to allow the support frame 910 to flex laterally to a higher degree between collar 115 and distal end 114.

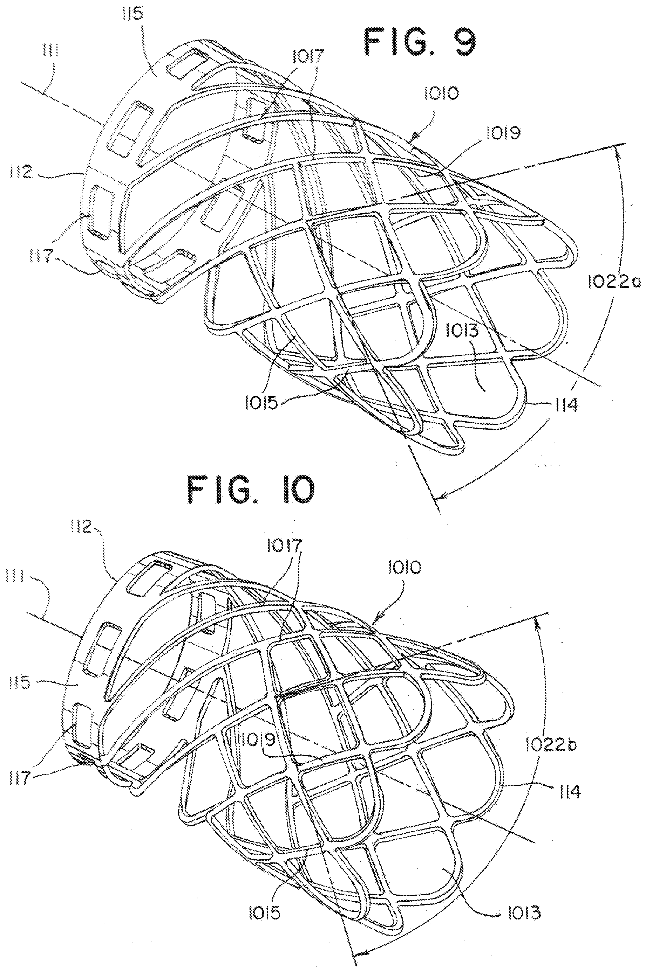

FIGS. 9 and 10 illustrate another example expandable support frame 1010 for use in a clot retrieval catheter tip 100. Support frame 1010 can include one or more features that are the same as or similar to those described above with respect to support frame 210. Further, the manufacturing of support frame 1010 can be the same as or similar to that of support frame 210, as discussed above. Support frame 1010 can also be manufactured to be electropolished, pickled to roughen the surface of support frame 1010, and/or heated to have an oxide layer finish.

A proximal end 112 of the support frame 1010 can include a wide collar 115 having a plurality collar cavities 117. Wide collar 115 is provided to give sufficient material for welding to a braided structure of an elongate body of a catheter delivery system by providing additional surface area to aid in welding the catheter tip 100 to a catheter system. Collar cavities 117 can be provided to allow materials to reflow during a lamination process to improve adhesion between the support frame 1010 and the elongate body of a catheter delivery system. The collar cavities 117 can also be effective to prevent excess material from bunching up over collar 115 in order to keep the outer diameter profile of catheter tip 100 low and even throughout the structure.

Support frame 1010 can include a longitudinal axis 111, a collapsed delivery configuration, and an expanded deployed configuration. The support frame 1010 can include a plurality of helical struts 1017 that extend from the collar 115. Helical struts 1017 can be angled with respect to the longitudinal axis 111 such that helical struts 1017 circle around the circumference of the support frame 1010. Extending from the helical struts 1017, support frame 1010 can include connector struts 1015. Connector struts 1015 and helical struts 1017 can intersect forming a lattice structure. The lattice structure of support frame 1010 can include lattice cells 1019. Lattice cells can include lattice cell angle 1022. FIG. 9 shows the lattice cell angle 1022a as the support frame 1010 is axially uncompressed, while FIG. 10 shows the support frame 1010 in an axially compressed state causing lattice cell angle 1022b to be greater than lattice cell angle 1022a.

In some examples, the pitch of the helix struts 1017 can be increased in the expanded deployed state to facilitate pushing the support frame 1010 through an outer guide sheath without causing the support frame 1010 to further expand as the catheter tip 100 is delivered to a treatment site. In some examples, the lattice cell angles 1022 can be reduced in the collapsed nondeployed state in order to prevent the support frame 1010 from expanding as it is delivered to a treatment site through a guide sheath.

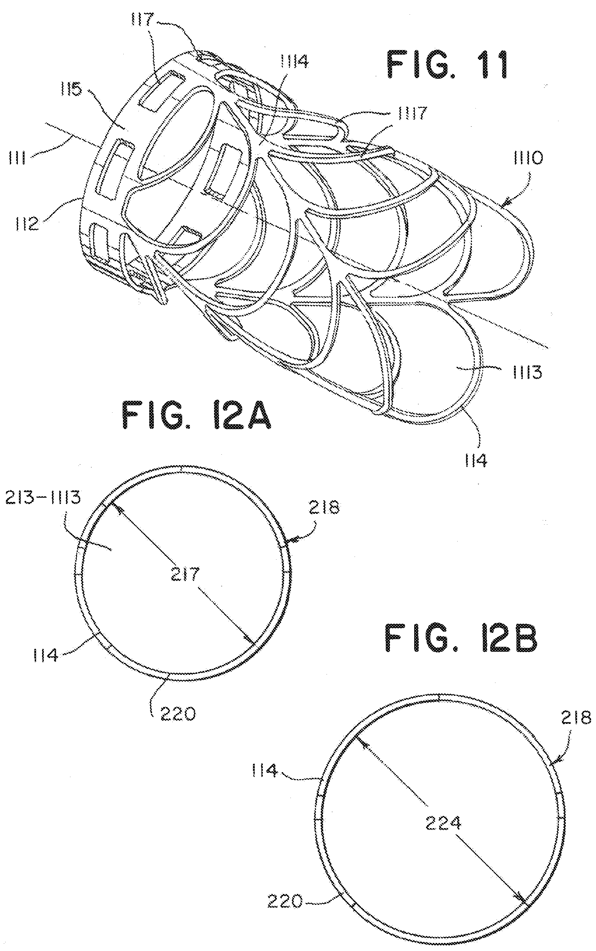

FIG. 11 illustrates another example expandable support frame 1110 for use in a clot retrieval catheter tip 100. Support frame 1110 can include one or more features that are the same as or similar to those described above with respect to support frame 210. Further, the manufacturing of support frame 1110 can be the same as or similar to that of support frame 210, as discussed above. Support frame 1110 can also be manufactured to be electropolished, pickled to roughen the surface of support frame 1110, and/or heated to have an oxide layer finish.

A proximal end 112 of the support frame 1110 can include a wide collar 115 having a plurality collar cavities 117. Wide collar 115 is provided to give sufficient material for welding to a braided structure of an elongate body of a catheter delivery system by providing additional surface area to aid in welding the catheter tip 100 to a catheter system. Collar cavities 117 can be provided to allow materials to reflow during a lamination process to improve adhesion between the support frame 1110 and the elongate body of a catheter delivery system. The collar cavities 117 can also be effective to prevent excess material from bunching up over collar 115 in order to keep the outer diameter profile of catheter tip 100 low and even throughout the structure.

Support frame 1110 can include one or more helical spines 1114 extending from collar 115. Helical spines 1114 can extend from collar 115 in a helical pattern around the circumference of the support frame 1110. Support frame 1110 is illustrated in FIG. 11 having four helical spines 1114, but in some examples support frame 1110 can have between one helical spine 1114 and 12 helical spines 1114. More helical spines 1114 of the same strut thickness can result in the tip being laterally stiffer and increase pushability of the catheter. Additionally, or alternatively, more helical spines 1114 can allow for thinner and/or shorter ribs 1117 and thus smaller openings between ribs 1117. The spacing between ribs 1117 preferably sufficiently small to mitigate likelihood that a membrane over the catheter tip can be aspirated to extend radially inwardly during use if using a very soft membrane.

Helical spines 1114 can be varied in pitch. For example, the helical pitch of helical spines 1114 can be increased to increase lateral stiffness and pushability of support frame 1110 to a treatment site. The helical pitch of helical spines 1114 can be decreased in order to reduce lateral flex. The pitch of the helical spines 1114 can be in the range of 0.5 to 50mm, more preferably in the range of 1 to 10mm, even more preferably 2 to 8mm. Higher pitch can result in more pushability but less flexibility. Extending from a respective helical spine 1114 can be one or more offset ribs 1117. The one or more offset ribs 1117 can extend around the circumference of support frame 1110 and can be substantially orthogonal to the longitudinal axis 111.

As shown in FIGS. 12A and 12B, the support frame 210 can include a collapsed inner diameter 217 (FIG. 12A) when in the collapsed delivery configuration, and a larger inner diameter 224 (FIG. 12B) in the expanded deployed configuration when the support frame 210 is placed in compression. The collapsed inner diameter 217 enables support frame 210 to fit within guide sheath 30 (FIG. 14A) when the catheter is being navigated through a vessel 12 toward a treatment site. As will be described further below with respect to FIGS. 15A-15C, when used in conjunction with an LST catheter, support frame 210 can maintain its collapsed inner diameter 217 as it exits the distal end 32 of the guide sheath 30, and then expand from the collapsed inner diameter 217 to the expanded inner diameter 224 when impinged by an ingested clot 40. Alternatively, when used in conjunction with a CSB catheter, as will be described further below with respect to FIGS. 14A-14C, support frame 210 can be heat set such that it has expanded inner diameter 224, greater than the collapsed inner diameter 217, as soon as support frame 210 exits the distal end 32 of the guide sheath 30. Used in conjunction with either an LST or CSB catheter, when support frame 210 is in its expanded deployed configuration (FIGS. 14B-14C and 15C), its maximum outer diameter can be less than an inner diameter 13 of the target vessel 12 at a treatment site, such that support frame 210 can advance distally toward clot 40 independently from and without sealing to the vessel 12.

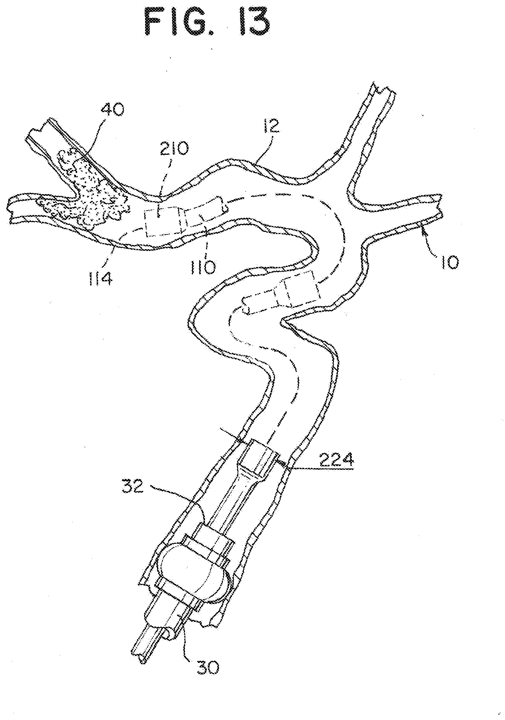

FIG. 13 illustrates a possible sequence for approaching an occlusive clot 40 using a large bore clot retrieval catheter 100 used in conjunction with the support frame designs disclosed herein. The clot 40 can be approached with the catheter 100 collapsed within a guide sheath 30 or other access catheter. When the vasculature 10 becomes too narrow and/or tortuous for further distal navigation with the guide sheath 30, the catheter 100 can be deployed for further independent travel distally. The catheter 100 can be highly flexible such that it is capable of navigating the M1 or other tortuous regions of the neurovascular system to reach an occlusive clot, and have an expanded outer diameter slightly less than that of the target vessel so the catheter is capable of distal navigation independently after deployment. In some examples, the catheter 100 can have an expanded outer diameter greater than that of the target vessel and the distal tip can be conformable to collapse in response to pressure applied from a smaller vessel having a smaller diameter than the expanded diameter as the distal tip is moved through the smaller vessel.

The clot retrieval catheter 100 can have a flexible elongate body 110 serving as a shaft with a large internal bore (which in some cases can be 0.080 inches (2.03mm) or larger) and a distal tip section having a collapsible support frame of the designs disclosed herein, such as support frame 210. The large bore helps the catheter to be delivered to a target site by a variety of methods. These can include over a guidewire, over a microcatheter, with a dilator/access tool, or by itself.

In many cases, the design of the tip can be configured so that the entire catheter 100 can be delivered through (and retrieved back through) common standard 6F sheaths/8F guides, which typically have inner lumens of less than 0.090 inches (2.3 mm). The tip can self-expand once advanced to an unconstrained position distal to the distal end 32 of the guide sheath 30. As the catheter can be deployed proximal of and then be advanced independently to a remote occlusion, the support frame of the tip is designed to be able to resist collapse from the forces of aspiration, have excellent lateral flexibility in both the expanded and collapsed states, and an atraumatic profile to prevent snagging on bifurcations in vessels.

FIGS. 14A-14C illustrate example treatment steps for using a clot retrieval catheter expandable support frame of the designs disclosed herein in conjunction with a CSB catheter, as described above. While FIGS. 14A-14C illustrate the use of support frame 1410, other support frames described herein (e.g., 210, 310, 410, 510, 610, 710, 810, 910, 1010, 1110) may be used in the same or similar series of treatment steps. FIG. 14A illustrates support frame 1410 inside a catheter shaft 110, and inside distal end 32 of guide sheath 30, as the catheter tip is moved through the target vessel 12 toward clot 40 (FIG. 14B). As shown, when inside the guide sheath 30 in its collapsed delivery configuration, support frame 1410 includes collapsed inner diameter 217.

FIG. 14B illustrates support frame 1410 transitioning to its expanded deployed configuration as it exits the distal end 32 of the guide sheath 30. As described above, when used in conjunction with a CSB catheter, support frame14210 can be heat set such that upon exiting the distal end 32 of the guide sheath 30, support frame 1410 can expand to an expanded inner diameter 224, which is greater than the collapsed inner diameter 217. The funnel profile 1416 of support frame 210 can also be symmetrical about the longitudinal axis 111. Alternatively, the funnel profile 1416 can be beveled. In some examples, when in its expanded deployed configuration, the maximum outer diameter of support frame 1410 can be less than an inner diameter 13 of target vessel 12 such that support frame 1410 can advance distally toward clot 40 independently from and without sealing to the vessel 12. In some examples, the maximum outer diameter of the support frame 1410 can be greater than the inner diameter 13 of the target vessel 12 and the support frame 1410 can be conformable to collapse in response to pressure applied from the smaller target vessel 12 as the support frame 1410 is moved through the target vessel 12 to the clot 40.

FIG. 14C illustrates a mouth 1413 of support frame 1410 engulfing a proximal portion of clot 40. As shown and discussed above, support frame 1410 can shorten axially when placed in compression against and/or around clot 40.

FIGS. 15A-15C illustrate example treatment steps for using a clot retrieval catheter expandable support frame of the designs disclosed herein in conjunction with an LST catheter, as described above. While FIGS. 11A-11C illustrate the use of support frame 1510, other support frames described herein (e.g., 210, 310, 410, 510, 610, 710, 810, 910, 1010, 1110) may be used in the same or similar series of treatment steps. FIG. 15A illustrates support frame 1510 inside a catheter shaft 110, and inside distal end 32 of guide sheath 30, as the catheter tip is moved through the target vessel 12 toward clot 40 (FIG. 15B). As shown, when inside the guide sheath 30 in its collapsed delivery configuration, support frame 1510 includes collapsed inner diameter 217.

FIG. 15B illustrates support frame 1510 exiting the distal end 32 of the guide sheath 30. As described above, when used in conjunction with an LST catheter, support frame 1510 can maintain its collapsed inner diameter 217 when exiting the distal end 32 of the guide sheath 30. As shown, the distal tip section 114 of support frame 1510 begins to align with a beveled mouth plane 1521. Alternatively, the distal support frame 1510 can have a symmetric profile.

FIG. 15C illustrates a mouth 1513 of support frame 210 engulfing a proximal portion of clot 40. As shown and discussed above, support frame 1510 can shorten axially when placed in compression against and/or around clot 40. Additionally, the funnel profile 1516 can be radially offset from the longitudinal axis 111. And similar to when support frame 1510 is used in conjunction with a CSB catheter (FIGS. 14A-14C), even when in its expanded deployed configuration, the maximum outer diameter of support frame 1510 can be less than an inner diameter 13 of target vessel 12 such that support frame 1510 can advance distally toward clot 40 independently from and without sealing to the vessel 12.

Catheters having a beveled mouth opening can be LST or CSB type designs. The beveled mouth opening provides a larger cross-sectional area that is in contact with the clot to increase the grip force compared to a circular, non-beveled opening. Frame designs that suit a beveled mouth opening include those in FIGs. 2, 3, 4, 5. The designs in FIGs. 1 and 6-11 may have a circular mouth or the membrane may be profiled to follow the profile of the distal struts.

The invention is not necessarily limited to the examples described, which can be varied in construction and detail. The terms "distal" and "proximal" are used throughout the preceding description and are meant to refer to positions and directions relative to a treating physician. As such, "distal" or distally" refer to a position distant to or a direction away from the physician. Similarly, "proximal" or "proximally" refer to a position near or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. As a result, not all possible combinations have been listed, and such variants are often apparent to those of skill in the art and are intended to be within the scope of the claims which follow. It is intended that each term contemplates its broadest meaning as understood by those skilled in the pertinent art and includes all technical equivalents that operate in a similar manner to accomplish a similar purpose without departing from the scope of the invention. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology. The scope of the invention is defined by the claims.

## Claims

1. A catheter tip (100) comprising:
a support frame (210) comprising:
a longitudinal axis (111);
a collapsed delivery configuration;
an expanded deployed configuration;
one or more profiled spine members (214) extending along the longitudinal axis connecting a distal most rib with a proximal most rib;
a plurality of ribs (215) extending from the one or more profiled spine members forming a circumference of the support frame;
one or more connector struts (216) each connecting respective adjacent ribs of the plurality of ribs, the one or more connector struts offset from the one or more profiled spine members, wherein each of the one or more connector struts are substantially parallel to the one or more profiled spine members; and
the support frame further comprising a collapsed inner diameter (217) in the collapsed delivery configuration and a larger expanded inner diameter (224) in the expanded deployed configuration when the support frame is placed in axial compression.

2. The catheter tip of claim 1, wherein the support frame is configured to expand from the collapsed inner diameter to the expanded inner diameter when impinged by an ingested clot (40).

3. The catheter tip of claim 1, wherein the support frame further comprises a proximal collar (115) at the proximal end of the support frame (112).

4. The catheter tip of claim 1, wherein the plurality of ribs each comprise a curvilinear, non-planar profile.

5. The catheter tip of claim 1, wherein each of the one or more connector struts are spaced approximately 90 degrees around the circumference of the support frame from each of the one or more profiled spine members.

6. The catheter tip of claim 1, wherein the one or more profiled spine members comprise two profiled spine members.

7. The catheter tip of claim 1, wherein two or more ribs of the plurality of ribs are unconnected by the one or more connector struts.

8. A catheter tip (100) comprising:
a support frame (310, 410, 510) comprising:
a longitudinal axis (111);
a collapsed delivery configuration;
an expanded deployed configuration;
a connector rib (315, 415, 515) extending around a circumference of the support frame in a first direction;
a plurality of offset ribs (317, 417, 517) extending from the connector rib around the circumference of the support frame in a second direction;
one or more connector members each connecting respective ribs of the plurality of offset ribs; and
the support frame further comprising a collapsed inner diameter (217) in the collapsed delivery configuration and a larger expanded inner diameter (224) in the expanded deployed configuration when the support frame is placed in axial compression,
wherein:
a) the one or more connector members further comprise one or more connector struts (316), wherein the one or more connector struts are substantially parallel to the longitudinal axis; or
b) the one or more connector members further comprise one or more compression cells (416, 516).

9. The catheter tip of claim 8a, wherein the one or more connector struts connect respective adjacent ribs of the plurality of offset ribs.

10. The catheter tip of claim 8b, wherein:
the one or more compression cells connect respective non-adjacent ribs of the plurality of offset ribs; or
the one or more compression cells comprise a generally diamond-shaped pattern; or
the one or more compression cells comprise a generally ovular-shaped pattern.

11. The catheter tip of claim 8, wherein the first direction and the second direction are approximately orthogonal.

12. The catheter tip of claim 8, wherein the support frame (610) further comprises:
a second connector rib (615b) extending from the proximal end of the support frame and around the circumference of the support frame; and
a second plurality of offset ribs (617b) extending from the second connector rib around the circumference of the support frame.

13. The catheter tip of claim 12, further comprising one or more connector struts (616) each connecting respective adjacent ribs of the second plurality of offset ribs.

## Patentansprüche

1. Katheterspitze (100), umfassend:
einen Stützrahmen (210), umfassend:
eine Längsachse (111);
eine zusammengeklappte Zuführkonfiguration;
eine expandierte entfaltete Konfiguration;
ein oder mehrere profilierte Rückgratelemente (214), die sich entlang der Längsachse erstrecken, die eine am distalsten gelegene Rippe mit einer am proximalsten gelegenen Rippe verbinden;
eine Vielzahl von Rippen (215), die sich von dem einen oder den mehreren profilierten Rückgratelementen erstrecken, die einen Umfang des Stützrahmens ausbilden;
einen oder mehrere Verbindungsstäbe (216), die jeweils entsprechende angrenzende Rippen der Vielzahl von Rippen verbinden, wobei der eine oder die mehreren Verbindungsstäbe zu dem einen oder den mehreren profilierten Rückgratelementen versetzt sind, wobei jeder des einen oder der mehreren Verbindungsstäbe im Wesentlichen parallel zu dem einen oder den mehreren profilierten Rückgratelementen ist; und
der Stützrahmen ferner umfassend einen zusammengeklappten Innendurchmesser (217) in der zusammengeklappten Zuführkonfiguration und einen größeren expandierten Innendurchmesser (224) in der expandierten entfalteten Konfiguration, wenn der Stützrahmen in axiale Kompression versetzt wird.

2. Katheterspitze nach Anspruch 1, wobei der Stützrahmen konfiguriert ist, um von dem zusammengeklappten Innendurchmesser zu dem expandierten Innendurchmesser zu expandieren, wenn er durch ein aufgenommenes Gerinnsel (40) beaufschlagt wird.

3. Katheterspitze nach Anspruch 1, wobei der Stützrahmen ferner einen proximalen Kragen (115) an dem proximalen Ende des Stützrahmens (112) umfasst.

4. Katheterspitze nach Anspruch 1, wobei die Vielzahl von Rippen jeweils ein krummliniges, nicht planares Profil umfasst.

5. Katheterspitze nach Anspruch 1, wobei jeder des einen oder der mehreren Verbindungsstäbe etwa 90 Grad um den Umfang des Stützrahmens herum von jedem des einen oder der mehreren profilierten Rückgratelemente beabstandet ist.

6. Katheterspitze nach Anspruch 1, wobei das eine oder die mehreren profilierten Rückgratelemente zwei profilierte Rückgratelemente umfassen.

7. Katheterspitze nach Anspruch 1, wobei zwei oder mehr Rippen der Vielzahl von Rippen nicht durch den einen oder die mehreren Verbindungsstäbe verbunden sind.

8. Katheterspitze (100), umfassend:
einen Stützrahmen (310, 410, 510), umfassend:
eine Längsachse (111);
eine zusammengeklappte Zuführkonfiguration;
eine expandierte entfaltete Konfiguration;
eine Verbindungsrippe (315, 415, 515), die sich um einen Umfang des Stützrahmens herum in einer ersten Richtung erstreckt;
eine Vielzahl von versetzten Rippen (317, 417, 517), die sich von der Verbindungsrippe um den Umfang des Stützrahmens herum in einer zweiten Richtung erstrecken;
ein oder mehrere Verbindungselemente, die jeweils entsprechende Rippen der Vielzahl von versetzten Rippen verbinden; und
der Stützrahmen ferner umfassend einen zusammengeklappten Innendurchmesser (217) in der zusammengeklappten Zuführkonfiguration und einen größeren expandierten Innendurchmesser (224) in der expandierten entfalteten Konfiguration, wenn der Stützrahmen in axiale Kompression versetzt wird,
wobei:
a) das eine oder die mehreren Verbindungselemente ferner einen oder mehrere Verbindungsstäbe (316) umfassen, wobei der eine oder die mehreren Verbindungsstäbe im Wesentlichen parallel zu der Längsachse sind; oder
b) das eine oder die mehreren Verbindungselemente ferner eine oder mehrere Kompressionszellen (416, 516) umfassen.

9. Katheterspitze nach Anspruch 8a, wobei der eine oder die mehreren Verbindungsstäbe jeweilige angrenzende Rippen der Vielzahl von versetzten Rippen verbinden.

10. Katheterspitze nach Anspruch 8b, wobei:
die eine oder die mehreren Kompressionszellen jeweilige nicht angrenzende Rippen der Vielzahl von versetzten Rippen verbinden; oder
die eine oder die mehreren Kompressionszellen ein grundsätzlich rautenförmiges Muster umfassen; oder die eine oder die mehreren Kompressionszellen ein grundsätzlich ovalförmiges Muster umfassen.

11. Katheterspitze nach Anspruch 8, wobei die erste Richtung und die zweite Richtung etwa orthogonal sind.

12. Katheterspitze nach Anspruch 8, wobei der Stützrahmen (610) ferner umfasst:
eine zweite Verbindungsrippe (615b), die sich von dem proximalen Ende des Stützrahmens und um den Umfang des Stützrahmens herum erstreckt; und
eine zweite Vielzahl von versetzten Rippen (617b), die sich von der zweiten Verbindungsrippe um den Umfang des Stützrahmens herum erstrecken.

13. Katheterspitze nach Anspruch 12, ferner umfassend einen oder mehrere Verbindungsstäbe (616), die jeweils entsprechende angrenzende Rippen der zweiten Vielzahl von versetzten Rippen verbinden.

## Revendications

1. Embout de cathéter (100), comprenant :
un cadre de support (210) comprenant :
un axe longitudinal (111) ;
une configuration d'administration repliée ;
une configuration déployée expansée ;
un ou plusieurs éléments de colonne profilés (214) s'étendant le long de l'axe longitudinal reliant une nervure la plus distale à une nervure la plus proximale ;
une pluralité de nervures (215) s'étendant à partir du ou des éléments de colonne profilés formant une circonférence du cadre de support ;
une ou plusieurs entretoises de liaison (216) reliant chacune des nervures adjacentes respectives de la pluralité de nervures, la ou les entretoises de liaison étant décalées par rapport au ou aux éléments de colonne profilés, dans lequel chacune parmi la ou les entretoises de liaison est sensiblement parallèle au ou aux éléments de colonne profilés ; et
le cadre de support comprenant en outre un diamètre interne replié (217) dans la configuration d'administration repliée et un diamètre interne expansé (224) plus grand dans la configuration déployée expansée lorsque le cadre de support est placé en compression axiale.

2. Embout de cathéter selon la revendication 1, dans lequel le cadre de support est conçu pour passer du diamètre interne replié au diamètre interne expansé lorsqu'il est heurté par un caillot (40) ingéré.

3. Embout de cathéter selon la revendication 1, dans lequel le cadre de support comprend en outre un collier proximal (115) au niveau de l'extrémité proximale (112) du cadre de support ;

4. Embout de cathéter selon la revendication 1, dans lequel la pluralité de nervures comprend chacune un profil curviligne non plan.

5. Embout de cathéter selon la revendication 1, dans lequel chacune parmi la ou les entretoises de liaison est espacée d'approximativement 90 degrés autour de la circonférence du cadre de support par rapport à chacun du ou des éléments de colonne profilés.

6. Embout de cathéter selon la revendication 1, dans lequel le ou les éléments de colonne profilés comprennent deux éléments de colonne profilés.

7. Embout de cathéter selon la revendication 1, dans lequel deux ou plusieurs nervures de la pluralité de nervures ne sont pas reliées par la ou les entretoises de liaison.

8. Embout de cathéter (100), comprenant :
un cadre de support (310, 410, 510) comprenant :
un axe longitudinal (111) ;
une configuration d'administration repliée ;
une configuration déployée expansée ;
une nervure de liaison (315, 415, 515) s'étendant autour d'une circonférence du cadre de support dans une première direction ;
une pluralité de nervures décalées (317, 417, 517) s'étendant à partir de la nervure de liaison autour de la circonférence du cadre de support dans une seconde direction ;
un ou plusieurs éléments de liaison reliant chacun des nervures respectives de la pluralité de nervures décalées ; et
le cadre de support comprenant en outre un diamètre interne replié (217) dans la configuration d'administration repliée et un diamètre interne expansé (224) plus grand dans la configuration déployée expansée lorsque le cadre de support est placé en compression axiale,
où :
a) le ou les éléments de liaison comprennent en outre une ou plusieurs entretoises de liaison (316), dans lequel la ou les entretoises de liaison sont sensiblement parallèles à l'axe longitudinal ; ou
b) le ou les éléments de liaison comprennent en outre une ou plusieurs cellules de compression (416, 516).

9. Embout de cathéter selon la revendication 8a, dans lequel la ou les entretoises de liaison relient des nervures adjacentes respectives de la pluralité de nervures décalées.

10. Embout de cathéter selon la revendication 8b, dans lequel :
la ou les cellules de compression relient des nervures non adjacentes respectives de la pluralité de nervures décalées ; ou
le ou les cellules de compression comprennent un motif généralement en forme de losange ; ou la ou les cellules de compression comprennent un motif généralement de forme ovale.

11. Embout de cathéter selon la revendication 8, dans lequel la première direction et la seconde direction sont approximativement orthogonales.

12. Embout de cathéter selon la revendication 8, dans lequel le cadre de support (610) comprend en outre :
une seconde nervure de liaison (615b) s'étendant à partir de l'extrémité proximale du cadre de support et autour de la circonférence du cadre de support ; et
une seconde pluralité de nervures décalées (617b) s'étendant à partir de la seconde nervure de liaison autour de la circonférence du cadre de support.

13. Embout de cathéter selon la revendication 12, comprenant en outre une ou plusieurs entretoises de liaison (616) reliant chacune des nervures adjacentes respectives de la seconde pluralité de nervures décalées.
